# EUROPEAN PATENT SPECIFICATION

(11) **EP 0 833 903 B1**
(45) Date of publication and mention of the grant of the patent: **25.08.2004**
(21) Application number: 96923275.0
(22) Date of filing: 07.06.1996
(51) Int. Cl.: C12N 15/11, C12N 7/04, A61K 39/215

(54) **MULTIVALENT BOVINE CORONAVIRUS VACCINE AND METHOD OF TREATING BOVINE CORONAVIRUS INFECTION**
MULTIVALENTER IMPSTOFF GEGEN BOVINES CORONAVIRUS UND METHODE ZUR BEHANDLUNG EINER INFEKTION MIT BOVINEM CORONAVIRUS
NOUVEAU VACCIN MULTIVALENT A BASE DE VIRUS CORONA BOVIN ET METHODE DE TRAITEMENT DE L'INFECTION AU VIRUS CORONA BOVIN

(30) Priority: 12.06.1995 US 489614
(43) Date of publication of application: 08.04.1998
(73) Proprietor: KANSAS STATE UNIVERSITY RESEARCH FOUNDATION, Manhattan, Kansas 66506 (US)
(72) Inventor: KAPIL, Sanjay, Manhattan, KS 66502 (US)
(74) Representative: UEXKÜLL & STOLBERG
(86) International application number: PCT/US1996/009979
(87) International publication number: WO 1996/041874

(56) References cited:
- US-A- 3 839 556
- US-A- 3 914 408
- MACINTYRE G ET AL: "Hyromycin B Therapy of a Murine Coronaviral Hepatitis" ANTIMICROBIAL AGENTS AND CHEMOTHERAPY, vol. 35, no. 10, October 1991, pages 2125-2127, XP002090023
- ARCHIVES VIROLOGY, Volume 140, issued 1995, TSUNEMITSU et al., "Antigenic and Biological Comparisons of Bovine Coronaviruses Derived from Neonatal Calf Diarrhea and Winter Dysentery of Adult Cattle", pages 1303-1311.
- VIROLOGY, Volume 106, issued 1980, BENEDETTO et al., "Inhibition of Animal Virus Production by Means of Translation Inhibitors Unable to Penetrate Normal Cells", pages 123-132.
- ANTIMICROBIAL AGENTS AND CHEMOTHERAPY, Volume 35, No. 10, issued October 1991, MACINTYRE et al., "Hygromycin B Therapy of a Murine Coronavirus Hepatitis", pages 2125-2127.
- ANTIMICROBIAL AGENTS AND CHEMOTHERAPY, Volume 35, No. 12, issued December 1991, MACINTYRE et al., "Hygromycin B Inhibits Synthesis of Murine Coronavirus RNA", pages 2630-2633.

## Description

### Background of the Invention

### 1. Field of the Invention

The present invention is directed to modified live vaccines for administration to cattle (especially newborn calves) in order to immunize the cattle against virulent wild-type bovine coronavirus infections (enteric and respiratory). The vaccines hereof contain low-passaged bovine coronavirus taken from the group consisting of Type II and Type III bovine coronavirus, and mixtures thereof, and may also contain the known Type I bovine coronavirus. The invention also pertains to a method of treating cattle with hygromycin B in sufficient quantities to suppress shedding of hygromycin B-sensitive bovine coronavirus in the feces of said cattle.

### 2. Description of the Prior Art

Bovine coronavirus (BCV) is an important cause of enterocolitis and respiratory tract infections in calves and adult cattle. In some instances, the diseases are referred to as calf diarrhea, calf scours or calf enteritis, and winter dysentery in adult cattle. Heretofore, only one serotype of BCV has been described, and modified live virus vaccines have been prepared by extensive passaging of the virulent virus. Such vaccines are described in U.S. Patent Nos. 3,839,556, 3,838,004 and 3,869,547.

However, in recent years, the known vaccines have proven to be clinically ineffective against many wild-type BCV infections, and indeed such infections are believed to cause a wide range of disease syndromes.

Another problem inherent in bovine coronavirus infections is the lack of effective treatment of cattle post-infection. A primary vector for spread of such infections results from shedding of virulent BCV in the feces of infected animals. To date, there has been no reported treatment for infected cattle which would suppress or eliminate such shedding.

There is accordingly a real and unsatisfied need in the art for new treatment modalities for cattle at risk for BCV infection, both in terms of a more effective anti-BCV vaccine and a treatment to suppress BCV shedding by infected cattle.

### Summary of the Invention

The present invention overcomes the problems outlined above, and provides new modified live BCV cattle vaccines which confer broader immunity against virulent wild-type BCV, as compared with existing vaccines. The invention is predicated upon the discovery of new antigenically distinct isolates of BCV which are referred to as Type II and Type III bovine coronavirus. These Type II and Type III coronavirus are preferably passaged up to about ten times in cell culture which supports the growth of the virus to create modified live vaccines. Excessive cell culture passages of the coronavirus should be avoided. The Type II and Type III BCV can be used individually as monovalent vaccines. More preferably, a multivalent vaccine comprising at least the Type II and Type III BCV is provided and such a vaccine may also contain the conventional Type I BCV (such as that deposited with the ATCC under Accession No. VR-874).

As used herein, Type I, Type II and Type III BCV isolates are defined in terms of the dilution inhibition titers developed in a one-way hemagglutination inhibition (HI) assay. That is, using this assay, Type I BCV has a dilution inhibition titer in the range of 1:512-1:4096; Type II BCV has a dilution inhibition titer of 1:32-1:256; and Type III has a inhibition dilution titer of 1:16 or less. The assay procedure to be used in generating the appropriate dilution inhibition titer data for determining an isolate type is fully described in Example 1.

In preferred forms, each dose of the modified live vaccines of the invention contain about 10⁴ to about 10⁸ plaque forming units (PFU) of bovine coronavirus, most preferably about 10⁶ PFU. In use, the modified live viruses of the invention are administered to calves, typically by oral-nasal inoculation. The most effective treatment is believed to be administration of the vaccine to newborn calves just after birth at least two hours before colostrum feeding.

In another aspect of the invention, it has been found that hygromycin B, a known feed additive for swine and poultry rations, can be administered to cattle as a treatment for chonic BCV infection in calves and adult cattle. Administration of hygromycin B serves to suppress or eliminate shedding of hygromycin B-sensitive BCV in the feces of cattle, thus minimizing a primary source of infection. Typically, hygromycin B is simply fed with the calf or cattle ration in amounts to at least suppress shedding of hygromycin B-sensitive BCV in the feces. A suitable dosage of hygromycin B would be from about 6-9 g of the drug per ton of cattle feed.

### Brief Description of the Drawing

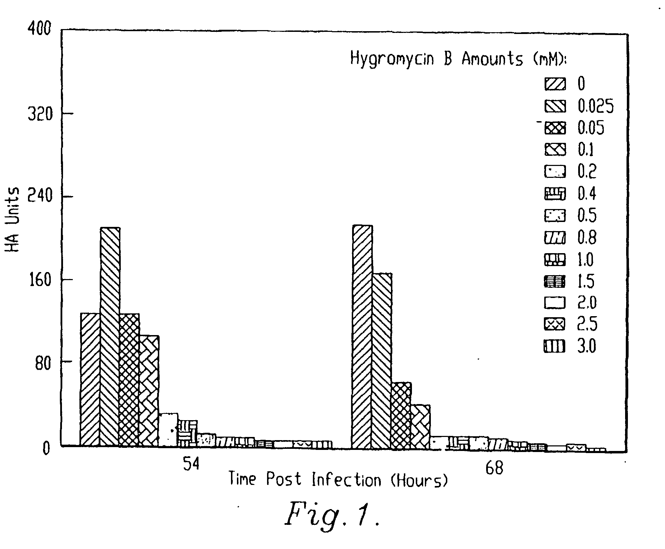

Figure 1 is a graphical representation setting forth the effect of hygromycin B upon cell culture viral replication of bovine coronavirus, as described in Example 4.

### Detailed Description of the Preferred Embodiments

The following examples describe the isolation and characterization of new BCV isolates in accordance with the invention, and moreover describes a manner of use thereof. It is be understood that these examples are set forth by way of illustration only, and nothing therein shall be taken as a limitation upon the overall scope of the invention.

### Example I

### Hemagglutination Assay (HA)/One-Way Hemagglutination Inhibition (HI) Assay Using 25 Fecal Suspensions Positive for Bovine Coronavirus

### Wisconsin Samples

A total of 20 bovine fecal samples were obtained from The Wisconsin Animal Health Laboratory, Madison, WI. Each of these samples had previously been determined by direct electron microscopy to contain bovine coronavirus, but were free of other known bovine virus. Each sample was diluted 1:10 v/v with calcium and magnesium ion-free PBS (CMF-PBS at pH 7.2).

Each sample was then centrifuged at 3000 rpm for 10 minutes and the clear supernatant was pipetted and saved. Each supernatant was then passed through a 0.45 micron, low protein-absorbing syringe filter. The filtrate of each sample was then subsequently assayed.

The first assay step was to determine the number of HAU (hemagglutination units) in each sample. Specifically, the BCV titer in each sample was determined using mouse erythrocytes suspended 0.5% v/v in PBS, pH 7.2, and containing 0.1% by weight BSA fraction V. First, successive two-fold dilutions (from 2-4096 fold) of each filtrate coronavirus sample were made using the PBS/BSA solution. Twenty-five microliters of each dilution were then placed in individual wells of a 96-well V-bottom microtiter plate, followed by the addition of an additional 25 microliters of the PBS/BSA solution into each well. Next, 25 microliters of the mouse erythrocyte suspension was added to each well. Thereupon, the side walls of the plate were tapped 4-5 times to insure mixing of the liquid fractions in each well. The plate was then covered and kept at 10°C for 90 minutes. The plate was then placed on a mirrored viewer. Each well was then visually examined to determine the hemagglutination end point titer for each sample of filtrate.

This titer data was used to determine the extent of dilution required to achieve, for each sample, a level of about 4-8 HAU/25 µl of filtrate sample. These dilutions were carried out in individual Eppendorf tubes, using the PBS/BSA solution.

Each standardized sample was then subjected to a hemagglutination inhibition (HI) assay. This assay is basically described by Sato et al., "Hemagglutination by Calf Diarrhea Coronavirus", *Vet. Microbiology,* **2** (1977) 83-87. The assay used in the present invention as a way to determine whether a given BCV is Type I, Type If or Type III virus is very similar to that described by Sato et al. In particular, HI assay used in connection with the present invention makes use of an anti-BCV serum such as the standard hyperimmune anti-BCV serum obtained from National Veterinary Services Laboratory, Ames, IA. A common serum obtained from this source is Lot No. 320BDV8801. The serum was first diluted (0.2 ml serum in 0.5 ml PBS) and heat inactivated by heating to 56°C and maintaining this temperature for 30 minutes. Next, the inactivated serum was treated by the addition of kaolin (0.4 ml of 25% kaolin), followed by centrifugation (15,000 g for 1 min.). The supernatant was then mixed with 0.2 ml of packed mouse erythrocyte per ml of supernatant. The treated serum was then maintained at 37°C for 1 hour. Thereafter, the treated serum was centrifuged (15,000 g for 1 min.) and the supernatant was used in the HI assay.

Increasing two-fold dilutions of the treated serum supernatant were then prepared and 25 µl of each dilution were placed in individual wells of a 96-well V-bottom microtiter plate. Twenty-five µl of each of the standardized filtrate samples were then added to each well followed by tapping and mixing. The plate was then incubated at 37°C for 1 hour. At this point, 25 µl of the previously described mouse erythrocyte suspension was added to each well, followed by tapping and mixing. A second incubation at 10°C was carried out for 90 minutes.

At the completion of the second incubation, the plate was visually examined to determine the extent of inhibition of hemagglutinating activity of each virus by the serum antibodies. Full inhibition is evidenced by the formation of a small "button" in the center of the wells. Lack of inhibition is shown by matting at the bottom of the wells.

Of the 20 starting samples, only four were fully inhibited by the treated serum, and it was concluded that these samples contained the known Type I BCV virus (W1-1.SK). Eight starting samples were not inhibited, two were inhibited up to 1:2 dilution, and two were inhibited up to 1:4 dilution, two were inhibited up to 1:8 dilution and one was diluted up to 1:16 dilution. These 15 samples were therefore BCV Type III virus. A single sample was inhibited up to 1:128 dilution, and this sample was deemed to include BCV Type II virus.

This experiment demonstrated that only a small number of the starting samples contained the Type I strain, whereas 15 of the samples contained the Type III strain. Accordingly, the known vaccines against the Type I strain would not confer immunogenicity against the Type III strains. It is postulated that the Types II and III BCV virus evolved from the originally isolated Type I virus under immunological pressure or may have evolved independently of the Type I virus.

### California Samples

Six California samples were tested in exactly the same manner as the Wisconsin samples. All six of the California samples were BCV Type III.

### Example 2

### Genetic Purification of BCV Virus Strains and Formation of Seed Stocks

Each of the 26 supernatants containing BCV as described in Example 1 were inoculated into cell culture.

Specifically, each cell culture contained human rectal tumor (HRT-18) cells obtained from Dr. David Benfield, University of South Dakota, Brookings, SD. These cells were screened and free of *Mycoplasma sp.,* bovine viral diarrhea virus (BVD) and any contaminating bacteria. These cells were plated in 25 cm² flasks and incubated at 37°C until the monolayer became confluent (usually 2-3 days). Each monolayer was then quickly washed with CMF-PBS containing 5 µl/ml of trypsin. 0.2 ml of each filtered and diluted supernatant of the 26 samples was then added to a respective treated cell culture. These flasks were rocked every 15 minutes during incubation at 37°C for 1 hour (original passage). Thereafter, 4-5 ml of MEM (minimal essential medium) was added to each flask. The MEM contained 5 µg/ml of both trypsin and pancreatin. Each flask was then further incubated at 37°C for a period of about 4-5 days with daily observation to determine cytopathic effect (CPE). CPE was determined by either rounding detachment or syncytium formation.

All of the 26 flasks were then tested in three different manners to determine and confirm the presence of BCV. First, each cell culture was tested by direct fluorescent antibody assay using FITC-labeled anti-BCV conjugate supplied by National Veterinary Services Laboratory, Ames, IA. The label directions with the conjugate were followed in performing the assay. Second, the amount of virus in each cell culture was determined using the HA assay described in Example 1. Third, each cell culture was checked for contaminating bovine viruses, using direct fluorescent antibody techniques, i.e., each culture was free of bovine rotavirus, IBR, BVD, bovine adenovirus, and bovine reovirus. Of the 26 cell cultures, 14 were confirmed to have propagated BCV; 8 of these cultures were from the Wisconsin samples; and 6 were from California.

The BCV-positive samples were harvested from each cell culture by three freeze-thaw cycles followed by centrifugation to pellet the cells. The cell-free supernatant is aliquoted (1 ml) and stored at -70°C.

In the next step, the 14 BCV isolates were plaque purified using the technique described in Kapil et al., Plaque Variations In Clinical Isolates of Bovine Coronavirus, J. Vet Diagn. Invest. 7 (1995) 538-539. Briefly, HRT-18 cells were plated in 6-well culture dishes and grown in MEM supplemented with 10% fetal calf serum (FCS), L-glutamine, penicillin and streptomycin. After 3-4 days propagation at 37°C, the resulting confluent monolayers were washed with CMF-PBS.

The 14 frozen harvested BCV samples were thawed just prior to use and diluted ten-fold in CMF-PBS. One hundred µl of each dilution (10⁻¹ to 10 ⁻⁶) were then placed in each well of the tissue culture dish. The culture dishes were incubated at 37°C for 1 hour, with rocking every 15 minutes. After incubation, each well was quickly washed with 1 ml of CMF-PBS. Next, 4 ml of MEM supplemented with 1% w/v agarose at 45°C was added to each well. The agarose was then allowed to solidify in each well, which usually took about 30 minutes at room temperature. The tissue culture plates were then inverted and incubated for up to three days at 37°C. The viral plaques appeared after about two days incubation. At the end of the incubation period, each viral plaque was singly harvested by pipette, about 50 µl of CMF-PBS was added, and the plaques were frozen at -70°C. There were about 69 resulting genetically pure plaques.

One plaque per viral isolate was selected for serial passage and propagation to form about 2 liters of virus. In particular, the selected plaques were thawed, diluted with 5-10 ml of the CMF-PBS, and about one-half ml of each dilution was added to individual 150 cm² tissue culture flasks. Incubation at 37°C over a period of 3 days followed. Four additional passages were carried out in the same fashion, resulting in a total of 6 passages, namely the original passage, plaque purification, and the four subsequent passages. At the end of the passage sequence, there was approximately 25 ml of passage 6 for each of the 14 isolates. In order to generate large volumes of each isolate, ½ ml of each isolate was placed in a respective 150 cm² flask. Incubation at 37°C followed as passage 7, and the collected quantities from each flask for each isolate were pooled. Each pooled isolate constituted a modified live vaccine.

A total of 11 monovalent modified live vaccines were successfully produced: two are Type I vaccines, one is a Type II vaccine, and eight are Type III vaccines.

### Example 3

### Use of Multivalent Modified Live Vaccines

The Type I, Type II and Type III isolates of Example 2 are administered *in vivo* by oral-nasal inoculation of approximately ½ ml of each isolate (each such dose containing about 10⁶ PFU of BCV). Such inoculation is given to colostrum-deprived calves immediately after birth. These calves are allowed colostrum two hours after birth. The inoculations confer immunity upon the calves at least against homologous types of BCV.

A multivalent vaccine is prepared by mixing equal quantities of selected Type I, Type II and Type III BCV isolates described in Example II. In like manner, a multivalent vaccine can be prepared using only Type II and Type III BCV isolates, if desired. The use of such multivalent vaccines is exactly as described above.

### Example 4

### Viral Suppression Using Hygromycin B

In this example, the effect of a feed additive drug, hygromycin B, on BCV in cell culture was tested. In a first set of experiments, three replicates each of individual cell cultures containing a Wisconsin isolate (W1-1.SK. a Type I BCV) were inoculated with varying amounts of hygromycin B and viral growth over time was monitored.

Each of the cell cultures contained HRT-18 cells treated as described in Example 2. Confluent monolayers in each culture were then inoculated with a dose containing about 32 HAU of the WI-1.SK isolate along with selected amounts of hygromycin B, ranging from zero (control) to 3 mM hygromycin B. Each of the cell cultures was then incubated at 37 °C for varying periods up to 90 hours. In order to determine the amount of virus in HAU over time, respective cell cultures were subjected to three freeze-thaw cycles and the HA analysis described previously was conducted on the supernatants.

The attached Figure illustrates the results of this test after 54 and 68 hours incubation following viral inoculation. As can be seen, those cultures inoculated with at least 0.4 mM hygromycin B exhibited very low, negligible viral growth; indeed, the results may simply be the effect of the original virus.

In a second set of experiments, 36 BCV isolates were tested to determine their susceptibility to hygromycin B using the cell culture assay described above, except that 1) replicate assays were not performed, 2) each culture contained either no hygromycin B (control) or hygromycin B at a concentration of 0.5 mM, and 3) each culture was incubated at 37°C for three days. The results of these experiments are shown in the following table:

| Isolate | -Hygromycin B (HAU) | +Hygromycin B (HAU) |
|---|---|---|
| MN-1 | 256 | <2 |
| MN-2 | 2048 | 8 |
| MN-5 | 4096 | 16 |
| MN-6 | 2048 | <2 |
| MN-7 | 2048 | 16 |
| MN-8 | 4096 | 16 |
| MN-9 | 4096 | 4096 |
| MN-10 | 256 | <2 |
| MN-11 | 4096 | 16 |
| MN-12 | 4096 | 4 |
| MN-13 | 512 | 128 |
| MN-15 | 32 | 4 |
| MN-16 | 512 | 8 |
| MN-17 | 512 | 16 |
| MN-19 | 2048 | 16 |
| MN-20 | 4096 | 4 |
| MN-21 | 2048 | 4 |
| MN-22 | 1024 | 8 |
| MN-23 | 128 | 32 |
| MN-24 | 512 | <2 |
| MN-25 | 256 | <2 |
| MN-26 | 512 | 8 |
| MN-27 | 1024 | 16 |
| MN-29 | 64 | 128 |
| MN-30 | 256 | 128 |
| MN-32 | 256 | 8 |
| MN-33 | 256 | 4 |
| MN-34 | 32 | <2 |
| MN-36 | 256 | 128 |
| MN-37 | 128 | <2 |
| MN-39 | 256 | 64 |
| MN-40 | 128 | 4096 |
| CA-1 | 256 | 64 |

These results demonstrate that hygromycin B is ineffective in inhibiting the in vitro replication of 4 of the 36 BCV isolates tested, namely the MN-9, MN-29, MN-30, and MN-36 isolates (those skilled in the art understand that the two-fold differences in HAU between cell culture assays with and without hygromycin B in these four tests are not statistically significant). The results of both sets of hygromycin B experiments demonstrate that hygromycin B inhibits BCV replication *in vitro,* inasmuch as the drug was effective against most BCV isolates tested irrespective of their antigenic composition and differences. This is strongly indicative that the drug will also suppress *in vivo* replication of the majority of BCV strains.

## Claims

1. An isolated bovine coronavirus, wherein the hemagglutinating activity of the bovine coronavirus (BCV) is fully inhibited at an antibody titer in the range of from about 1:1 to 1:16 and is uninhibited at a titer of about 1:32 and above, in the one-way mouse erythrocyte hemagglutination inhibition assay of Example I using standard National Veterinary Service Laboratory hyperimmune anti-BCV serum.

2. A vaccine comprising a bovine coronavirus, wherein the hemagglutinating activity of the bovine coronavirus (BCV) is fully inhibited at an antibody titer in the range of from about 1:1 to 1:16 and is uninhibited at a titer of about 1:32 and above, in the one-way mouse erythrocyte hemagglutination inhibition assay of Example I using standard National Veterinary Service Laboratory hyperimmune anti-BCV serum.

3. The vaccine of claim 2, wherein a dose of the vaccine contains from 10⁴ to 10⁸ PFU.

4. The vaccine of claim 2, wherein the virus has been passaged up to 10 times in cell culture which supports growth of the virus.

5. Use of an isolated bovine coronavirus, wherein the hemagglutinating activity of the bovine coronavirus (BCV) is fully inhibited at an antibody titer in the range of from about 1:1-1:16 and is uninhibited at a titer of about 1:32 and above, in the one-way mouse erythrocyte hemagglutination inhibition assay of Example I using standard National Veterinary Service Laboratory hyperimmune anti-BCV serum, for preparing a vaccine for immunizing a calf against infection by a bovine coronavirus.

6. The use of claim 5, wherein a dose of the vaccine contains from 10⁴ to 10⁸ PFU.

7. The use of claim 5, wherein the vaccine is for oral or nasal administration.

8. The use of claim 5, wherein the calf is colostrum-deprived.

9. The use of claim 5, comprising the use of a sufficient amount of hygromycin B to suppress shedding of bovine coronavirus into the feces of the calf.

## Patentansprüche

1. Ein isoliertes bovines Coronavirus, wobei die hämoglutinierende Aktivität des bovinen Coronavirus (BCV) bei Antikörpertitern von ungefähr 1:1 bis 1:16 vollständig unterdrückt ist und bei Titern von ungefähr 1:32 und höher nicht unterdrückt ist im einseitig ausgerichteten Maus-Erythrozyt-Hämoglutinierung-Inhibierungsassay des Beispiels 1 unter Verwendung von standard hyperimmun anti-BCV Serum des National Veterinary Service Laboratory.

2. Ein Impfstoff, das ein bovines Coronavirus enthält, wobei die hämoglutinierende Aktivität des bovinen Coronavirus (BCV) bei Antikörpertitern von ungefähr 1:1 bis 1:16 vollständig unterdrückt ist und bei Titern von ungefähr 1:32 und höher nicht unterdrückt ist im einseitig ausgerichteten Maus-Erythrozyt-Hämoglutinierung-Inhibierungsassay des Beispiels 1 unter Verwendung von standard hyperimmun anti-BCV Serum des National Veterinary Service Laboratory.

3. Der Impfstoff nach Anspruch 2, wobei eine Impfstoffdosis 10⁴ bis 10⁸ PFU enthält.

4. Der Impfstoff nach Anspruch 2, wobei das Virus bis zu 10 mal in einer Zellkultur passagiert wurde, die das Virenwachstum unterstützt.

5. Eine Verwendung eines isolierten bovinen Coronavirus, wobei die hämoglutinierende Aktivität des bovinen Coronavirus (BCV) bei Antikörpertitern von ungefähr 1:1 bis 1:16 vollständig unterdrückt ist und bei Titern von ungefähr 1:32 und höher nicht unterdrückt ist im einseitig ausgerichteten Maus-Erythrozyt-Hämoglutinierung-Inhibierungsassay des Beispiels 1 unter Verwendung von standard hyperimmun anti-BCV Serum des National Veterinary Service Laboratory, um einen Impfstoff zur Immunisierung eines Kalbes gegen die Infektion durch ein bovines Coronavirus herzustellen.

6. Die Verwendung nach Anspruch 5, wobei eine Impfstoffdosis 10⁴ bis 10⁸ PFU enthält.

7. Die Verwendung nach Anspruch 5, wobei der Impfstoff zur oralen oder nasalen Verabreichung bestimmt ist.

8. Die Verwendung nach Anspruch 5, wobei das Kalb kein Kolostrum bekommt.

9. Die Verwendung nach Anspruch 5, die die Verwendung einer ausreichenden Menge von Hygromycin B enthält, um die Ausscheidung des bovinen Coronaviruses in die Faeces des Kalbes zu unterdrücken.

## Revendications

1. Coronavirus bovin isolé, dans lequel l'activité hémagglutinante du coronavirus bovin (BCV) est totalement inhibée à un titre d'anticorps compris dans une gamme de 1 : 1 à 1 : 16 et n'est pas inhibée à un titre d'environ 1 : 32 et plus, dans le test d'inhibition d'hémagglutination des érythrocytes murins unidirectionnel de l'exemple 1, en utilisant un sérum anti-BCV hyperimmun de référence du National Veterinary Service Laboratory.

2. Vaccin comprenant un coronavirus bovin, dans lequel l'activité hémagglutinante du coronavirus bovin (BCV) est totalement inhibée à un titre d'anticorps compris dans une gamme de 1 : 1 à 1 : 16 et n'est pas inhibée à un titre d'environ 1 : 32 et plus, dans le test d'inhibition d'hémagglutination des érythrocytes murins unidirectionnel de l'exemple 1, en utilisant un sérum anti-BCV hyperimmun de référence du National Veterinary Service Laboratory.

3. Vaccin selon la revendication 2, où une dose du vaccin contient de 10⁴ à 10⁸ UFP.

4. Vaccin selon la revendication 2 dans lequel le virus a fait l'objet de jusqu'à dix passages en culture de cellules permettant la prolifération virale.

5. Utilisation d'un Coronavirus bovin isolé, dans lequel l'activité hémagglutinante du coronavirus bovin (BCV) est totalement inhibée à un titre d'anticorps compris dans une gamme de 1 : 1 à 1 : 16 et n'est pas inhibée à un titre d'environ 1 : 32 et plus, dans le test d'inhibition d'hémagglutination des érythrocytes murins unidirectionnel de l'exemple 1, en utilisant un sérum anti-BCV hyperimmun de référence du National Veterinary Service Laboratory pour la préparation d'un vaccin destiné à immuniser un veau vis-à-vis de l'infection à coronavirus bovin.

6. Utilisation selon la revendication 5, une dose de vaccin contenant de 10⁴ à 10⁸ UFP.

7. Utilisation selon la revendication 5, le vaccin étant destiné à une administration orale ou nasale.

8. Utilisation selon la revendication 5, où le veau est privé de colostrum.

9. Utilisation selon la revendication 5, comprenant l'utilisation d'une quantité suffisante d'hygromycine B, pour supprimer l'excrétion de coronavirus bovin dans les matières fécales du veau.
